# EUROPEAN PATENT APPLICATION

(11) **EP 3 539 414 A1**
(43) Date of publication of application: **18.09.2019**
(21) Application number: 18175760.0
(22) Date of filing: 04.06.2018
(51) Int. Cl.: A45D 44/00, B65D 75/58, A45D 37/00

(54) **MASK PACK**

(30) Priority: 13.03.2018 KR 20180029182
(71) Applicant: The Oozoo Co., Ltd., Seoul (KR)
(72) Inventor: LEE, Ha Jun, 07064 Seoul (KR)
(74) Representative: Meissner Bolte Partnerschaft mbB

(57) **Abstract**

A mask pack according to the present invention includes: a pouch keeping a mask sheet and having a hole at a side; a blister including a body formed in a plate shape, having a storing portion protruding upward from the center portion to the outside through the hole of the pouch to keep a cosmetic liquid, and having other portion fixed to an inner side of the pouch in the pouch, except for the storing portion, and a cover covering a bottom of the body to seal the storing portion and being torn or removed by external force so that the cosmetic liquid is discharged into the pouch and soaks the mask sheet.

## Description

### BACKGROUND OF THE INVENTION

### Field of the Invention

The present invention relates to a mask pack and, more particularly, a mask pack that keeps separately a skincare agent and a facial mask sheet.

### Description of the Related Art

Recently, not only women, but men have interest in skincare to care their impressions. In particular, their interest in skin anti-aging for keeping the skin elastic and clean is increasing, so various products for skincare have been released and facial masks are generally used as a method for facial skincare.

A facial mask, which is a kind of skincare product, is formed by soaking a sheet with a cosmetic or a liquid with a skincare effect and is applied to the face so that the cosmetic, etc. directly permeates into the skin. Facial masks are sealed in thin containers having an internal storing space and users open the containers to use the facial mask.

In detail, facial masks in the related art are released in various shapes such as a circle or a facial shape by compressing a base such as a non-woven fabric to be put on the face so that a user can easily use them, and are packed by the piece. These facial masks are formed by soaking a base such as non-woven fabric with a substance having predetermined viscosity or applying the substance to the base. Recently, natural liquids and various components containing vitamins that help skincare are added to the bases to enhance the functionality of facial masks.

However, some of functional substances, such as vitamin C, are degenerated or reduced in their functionality when they are exposed to components containing water for a long time, and when different components are mixed for a long period of time, they may be degenerated. Further, the natural liquids that help skincare are generally natural extracts obtained in the process of fermenting and ripening, so the liquids may easily oxidize or decompose in summer or at high indoor temperature.

In particular, fiber textures such as pure cotton or polyester non-woven fabric are generally used for facial mask sheets, but when the facial mask sheets are soaked with liquids such as natural extracts, degeneration of the natural liquids may be accelerated. For this reason, it is required to develop a technology that can separately keep a liquid for a facial mask and a facial mask sheet and enable the facial mask sheet and the liquid for a facial mask to be simply mixed.

The foregoing is intended merely to aid in the understanding of the background of the present invention, and is not intended to mean that the present invention falls within the purview of the related art that is already known to those skilled in the art.

### SUMMARY OF THE INVENTION

The present invention has been made in an effort to solve the problems and an object of the present invention is to provide a mask pack that allows for a user to use a fresher and more sanitary facial mask sheet and accordingly can improve a skincare effect by separately keeping a liquid for a facial mask, which has been kept with a facial mask sheet, in a separate container such that a user can simply mix the liquid for a facial mask with a facial mask sheet when he/she uses a facial mask.

In order to achieve the objects of the present invention, a mask pack includes: a pouch keeping a mask sheet and having a hole at a side; a blister including a body formed in a plate shape, having a storing portion protruding upward from the center portion to the outside through the hole of the pouch to keep a cosmetic liquid, and having other portion fixed to an inner side of the pouch in the pouch, except for the storing portion, and a cover covering a bottom of the body to seal the storing portion and being torn or removed by external force so that the cosmetic liquid is discharged into the pouch and soaks the mask sheet.

The cover may be made of a high molecular compound.

The cover may seal the storing portion by being bonded to the bottom of the body.

The cover may be formed with a predetermined thickness and may have a groove having a predetermined length in a width direction.

The groove may be formed on a side, which is exposed to an inside of the pouch, of the cover.

When external force over a predetermined magnitude is applied to both ends of the body such that the blister is bent, the groove on the cover may be torn and the cosmetic liquid in the storing portion may be discharged into the pouch, so the mask sheet may be soaked with the cosmetic liquid.

The cover may have: a sealing portion sealing the storing portion by covering the bottom of the body; and a pull extending from an end of the sealing portion.

The sealing portion may seal the storing portion by being bonded to the bottom of the body.

The pull may extend from an end of the sealing portion longer than a maximum length of the body.

A first end of the pull may be bonded to the bottom of the body and a second end of the pull may be folded to a second end of the sealing portion from the first end bonded to the bottom of the body.

A slit through which the second end of the pull is disposed may be formed through a side of the pouch.

When the blister is fixed to the pouch, the second end of the pull may be exposed to the outside through the slit of the pouch.

A slit through which the second end of the pull is disposed may be formed through a side of the body.

When the hole of the pouch is larger than the storing portion of the blister and the blister is fixed to the pouch, the second end of the pull may be exposed to the outside trough the slit of the body and the hole of the pouch.

When external force over a predetermined magnitude is applied to the second end of the pull, the sealing portion sealing the storing portion by being bonded to the bottom of the body may be removed, so the cosmetic liquid in the storing portion may be discharged into the pouch and may permeate into the mask sheet.

The blister may have: a first adhesive section bonded to the cover, as a section of an edge of the storing portion, to be detached when pressure over a predetermined magnitude is applied to the storing portion; a second adhesive section bonded to the cover more strongly than the first adhesive portion, as the other section except for the first adhesive section of the edge of the storing portion; a non-adhesive section formed outside the first bonding section and having a predetermined area where the body and the cover are not bonded to each other; a third adhesive section bonding the body and the cover to each other outside the edge of the storing portion and the non-adhesive section; and a crease formed on the cover at the non-adhesive section.

Edges of a first end and a second end of the storing portion may be formed in an arc shape and the first adhesive section has the same curvature as the edge of the second end of the storing portion.

The body and the cover may be physically coupled to each other at the first adhesive section by an embossing structure.

The body and the cover may be chemically bonded to each other at the second adhesive section by an adhesive.

The non-adhesive section may have an inner end, an outer end, and a both sides.

The second adhesive section may extend to the both sides of the non-adhesive section.

The inner end may be spaced a predetermined distance from the edge of a second part of the storing portion, the outer end may be spaced a predetermined distance from the inner end, and the both sides may be formed inside the second adhesive section.

The crease may be formed in a curve shape convexly toward the outer end of the non-adhesive section at a predetermined distance in the non-adhesive section from the first adhesive section.

When pressure over a predetermined magnitude is applied to the storing portion, the first adhesive section is detached, and the cosmetic liquid in the storing portion is discharged, whereby the cover may be torn downward at the crease by pressure of the cosmetic liquid, a discharge hole may be formed at the torn portion, and the cosmetic liquid may be discharged into the pouch through the discharge hole and permeates into the mask sheet.

According to the mask pack of the present invention, a cosmetic liquid is separately kept in the separate container such that a user can simply mix the cosmetic liquid and the mask sheet to use a facial mask, so the user can use a fresher and more sanitary facial mask and accordingly skincare effect can be improved.

### BRIEF DESCRIPTION OF THE DRAWINGS

The above and other objects, features and other advantages of the present invention will be more clearly understood from the following detailed description when taken in conjunction with the accompanying drawings, in which:
FIG. 1 is a view schematically showing the configuration of a mask pack according to the present invention;
FIG. 2 is a perspective view of a blister of a mask pack according to a first embodiment of the present invention;
FIG. 3 is a view when the blister is inserted in a pouch of the mask pack according to the first embodiment;
FIG. 4 is a cross-sectional view taken along line A-A' of FIG. 3;
FIG. 5 is a view showing an example of actually using the mask pack according to the present invention;
FIG. 6 is a perspective view showing a blister separated from a mask pack according to a second embodiment of the present invention;
FIG. 7 is a view showing the blister with a body and a cover combined in the mask pack according to the second embodiment of the present invention;
FIG. 8 is a view showing the blister inserted in a pouch of the mask pack according to the second embodiment of the present invention;
FIG. 9 is a cross-sectional view taken along line B-B' of FIG. 8;
FIG. 10 is a view showing an example of actually using the mask pack according to the second embodiment of the present invention;
FIG. 11 is a perspective view showing a blister of a mask pack according to a third embodiment of the present invention;
FIG. 12 is a plan view of a blister of a mask pack according to the third embodiment of the present invention;
FIG. 13 is a view showing the blister inserted in a pouch of the mask pack according to the third embodiment of the present invention;
FIG. 14 is a cross-sectional view taken along line C-C' of FIG. 13; and
FIG. 15 is a view showing an example of actually using the mask pack according to the third embodiment of the present invention.

### DETAILED DESCRIPTION OF THE INVENTION

A mask pack according to exemplary embodiments of the present invention is described hereafter with reference to the accompanying drawings.

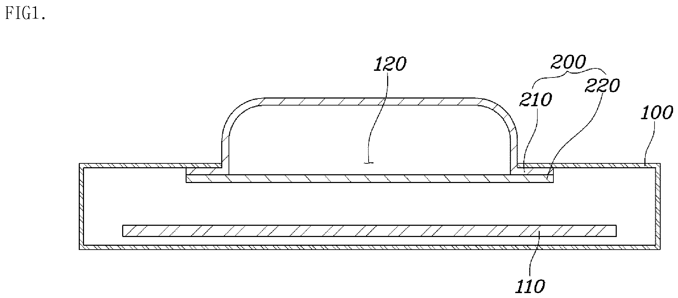

FIG. 1 is a view schematically showing the configuration of a mask pack according to the present invention. As shown in FIG. 1, a mask pack according to the present invention includes: a pouch 100 keeping a mask sheet 110 and having a hole 120 at a side; a blister 200 including a body 210 formed in a plate shape, having a storing portion 211 protruding upward from the center portion to the outside through the hole 120 of the pouch 100 to keep a cosmetic liquid, and having other portion fixed to the inner side of the pouch 100 in the pouch 100, except for the storing portion 211, and a cover 220 covering the bottom of the body 210 to seal the storing portion 211 and being torn or removed by external force so that the cosmetic liquid is discharged into the pouch 100 and permeates into the mask sheet 110.

According to the mask pack of the present invention, a cosmetic liquid is separately kept in the separate blister such that a user can mix the cosmetic liquid and the mask sheet to use a facial mask by simply cutting or removing the cover by applying force to the storing portion. Accordingly, the user can use a fresher and more sanitary facial mask, so the skincare effect can be improved.

Mask packs according to various embodiments are described hereafter in detail with reference to the accompanying drawings.

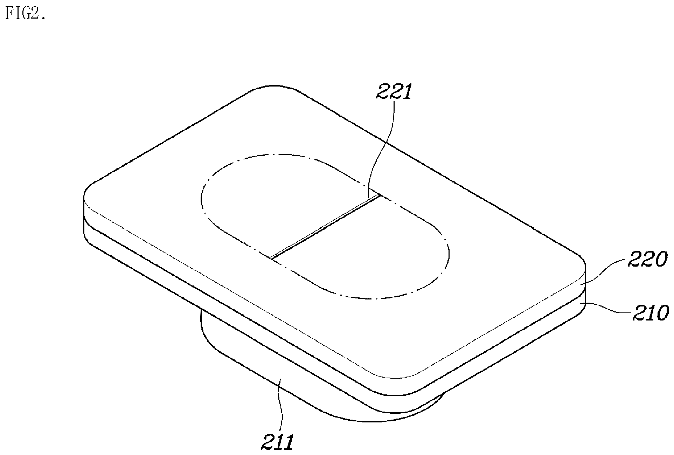

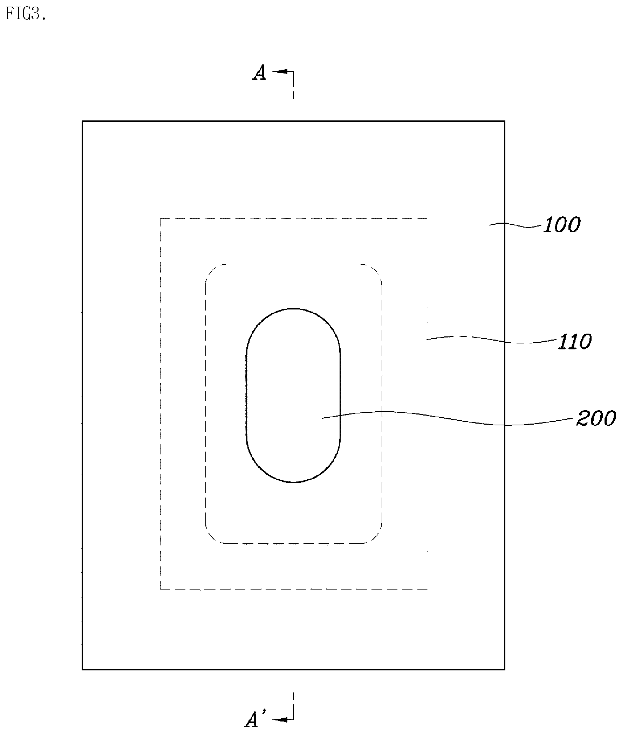

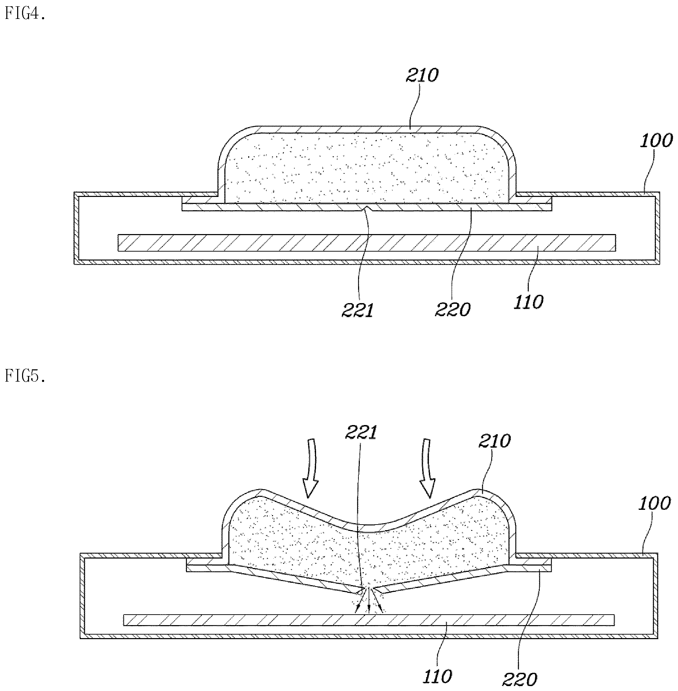

FIG. 2 is a perspective view of a blister of a mask pack according to a first embodiment of the present invention, FIG. 3 is a view when the blister is inserted in a pouch of the mask pack according to the first embodiment, FIG. 4 is a cross-sectional view taken along line A-A' of FIG. 3, and FIG. 5 is a view showing an example of actually using the mask pack according to the present invention. In the perspective view of FIG. 2, the blister has been turned over to show a groove formed on the cover of the blister.

The mask pack according to the first embodiment of the present invention may include a pouch 100 that keeps a mask sheet 110 and a blister 200 that includes a body 210 and a cover 220.

In detail, the body 210 is formed in a plate shape, has a storing portion 211 protruding upward from the center portion to the outside through a hole 120 of the pouch 100 to keep a cosmetic liquid, and has other portion fixed to the inner side of the pouch 100 in the pouch 100, except for the storing portion 211. The portion of the body 210 except for the storing portion 211 may be chemically attached to the inner side of the pouch 100 by an adhesive etc. That is, the portion of the body 210 except for the storing portion 211 may be chemically completely bonded to the inner side of the pouch 100 by an adhesive etc.

The cover 220 can seal the storing portion 211 by covering the bottom of the body 211. The cover 220 may completely seal the storing portion 211 by being chemically attached to the bottom of the body 210 by an adhesive etc., thereby being able to prevent leakage of a cosmetic liquid in the storing portion 211.

The cover 220 may be made of a high molecular compound including plastic. The cover 220 may have a predetermined thickness, and as show in FIG. 2, may have a groove 221 having a predetermined length in the width direction. The groove 221 is formed shallower than the thickness of the cover 220, and as show in FIG. 3, it may be formed on the side, which is exposed to the inside of the pouch 100, of the cover 220.

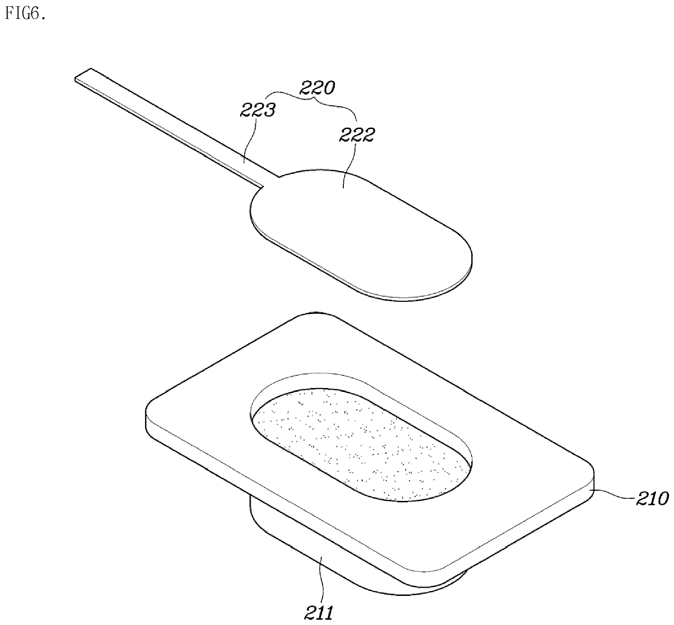

Referring to FIG. 5, an example of actually using the mask pack according to the first embodiment of the present invention is described. When external force over a predetermined magnitude is applied to both ends of the body 210 such that the blister 200 is bent, the groove on the cover 220 is torn and the cosmetic liquid in the storing portion 211 is discharged into the pouch 100, so the mask sheet 110 can be soaked with the cosmetic liquid. According to the mask pack of the first embodiment of the present invention, as described above, a cosmetic liquid is separately kept in the separate blister, and when external force over a predetermined magnitude is applied to both ends of the body such that the blister is bent to use a facial mask, the groove on the cover is torn and the cosmetic liquid in the storing portion is discharged. Therefore, a user can simply mix and use the cosmetic liquid and the mask sheet and use a fresher and more sanitary facial mask, and accordingly, the skincare effect can be improved. Hereinafter, a mask pack according to a second embodiment of the present invention will be described in detail with reference to FIGS. 6 to 10.

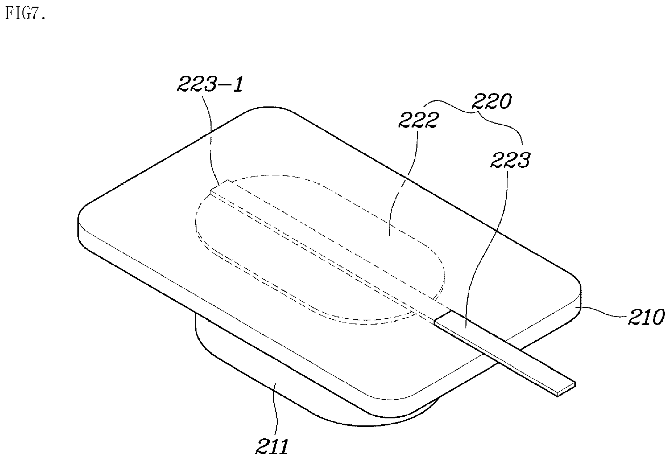

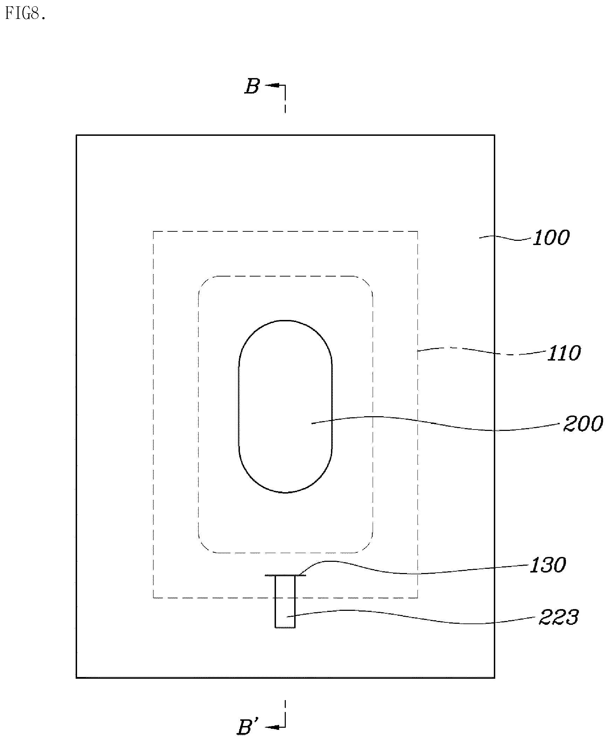

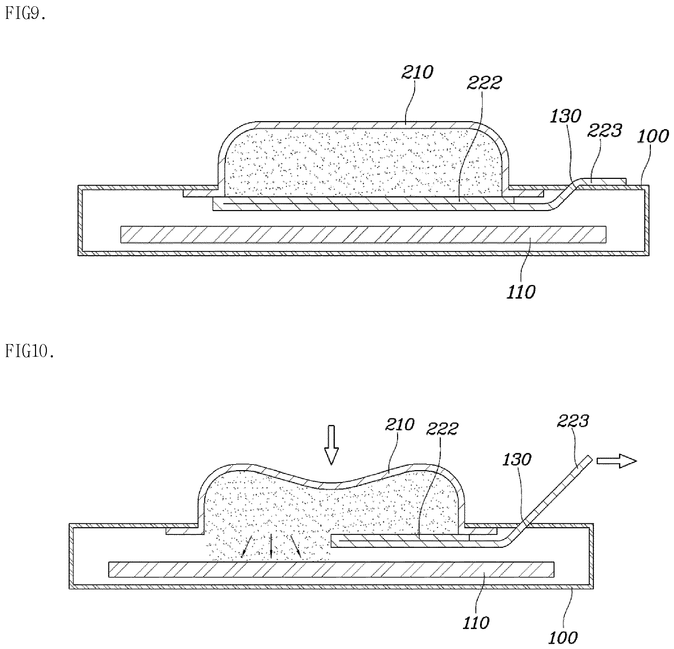

FIG. 6 is a perspective view of a mask pack according to the second embodiment of the present invention with a blister separated, FIG. 7 is a view showing the blister with a body and a cover combined in the mask pack according to the second embodiment of the present invention, FIG. 8 is a view showing the blister inserted in a pouch of the mask pack according to the second embodiment of the present invention, FIG. 9 is a cross-sectional view taken along line B-B' of FIG. 8, and FIG. 10 is a view showing an example of actually using the mask pack according to the second embodiment of the present invention.

The mask pack according to the second embodiment of the present invention may include a pouch 100 that keeps a mask sheet 110 and a blister 200 that includes a body 210 and a cover 220.

In detail, the body 210 is formed in a plate shape, has a storing portion 211 protruding upward from the center portion to the outside through a hole 120 of the pouch 100 to keep a cosmetic liquid, and has other portion fixed to the inner side of the pouch 100 in the pouch 100, except for the storing portion 211. The portion of the body 210 except for the storing portion 211 may be chemically coupled to the inner side of the pouch 100 by an adhesive etc. That is, the portion of the body 210 except for the storing portion 211 may be chemically completely bonded to the inner side of the pouch 100 by an adhesive etc.

The cover 220, as shown in FIG. 6, may have a sealing portion 222 that seals the storing portion 211 by covering the bottom of the body 210 and a pull 223 extending from an end of the sealing portion 222. The sealing portion 222 may completely seal the storing portion 211 by being chemically attached to the bottom of the body 210 by an adhesive etc., thereby being able to prevent leakage of a cosmetic liquid in the storing portion 211. The pull 223 may extend from a first end of the sealing portion 222 longer than the maximum length of the body 210, in which the maximum length of the body 210 may be the width of the body 210.

Referring to FIG. 7, when the sealing portion 222 is bonded to the bottom of the body 210, a first end 223-1 of the pull 223 may be bonded to the bottom of the body 210 and a second end of the pull 223 may be folded to a second end of the sealing portion 222 from the first end 223-1 bonded to the bottom of the body 210. Referring to FIG. 8, a slit 130 may be formed through a side of the pouch 110, so when the blister 200 is fixed to the pouch 100, the second end, which is folded to the second end of the sealing portion 222, of the pull 223 can be exposed to the outside through the slit formed through a side of the pouch 100.

An example of actually using the mask pack according to the second embodiment of the present invention is described with reference to FIG. 10. When external force over a predetermined magnitude is applied to the second end of the pull 223 exposed outside through the slit 130 of the pouch 100, the sealing portion 222 sealing the storing portion 211 by being bonded to the bottom of the body 210 is removed, so a cosmetic liquid in the storing portion 211 can be discharged into the pouch 100 and can permeate into the mask sheet 110. According to the mask pack of the second embodiment of the present invention, as described above, a cosmetic liquid is separately kept in the separate blister, and when the pull exposed outside the pouch is pulled by force over a predetermined magnitude to use a facial mask, the sealing portion sealing the storing portion by being bonded to the bottom of the body is removed, so the cosmetic liquid in the storing portion is discharged. Therefore, a user can simply mix and use the cosmetic liquid and the mask sheet and use a fresher and more sanitary facial mask, and accordingly, the skincare effect can be improved.

Meanwhile, depending on embodiments, a slit (not shown) through which the second end of the pull 223 is disposed may be formed through a side of the body 210 and the hole 120 of the pouch 100 may be formed larger than the storing portion 211 of the blister 200. That is, when the blister 200 is fixed to the pouch 100, a side of the body 210 may be exposed to the outside through the hole 120 of the pouch 100. Further, in this case, when the blister 200 is fixed to the pouch 100, the second end of the pull 223 may be exposed to the outside through the hole 120 of the pouch 100 and the slit (not shown) formed at the body 210. In this case, the method of using the mask pack is the same as that described with reference to FIG. 10.

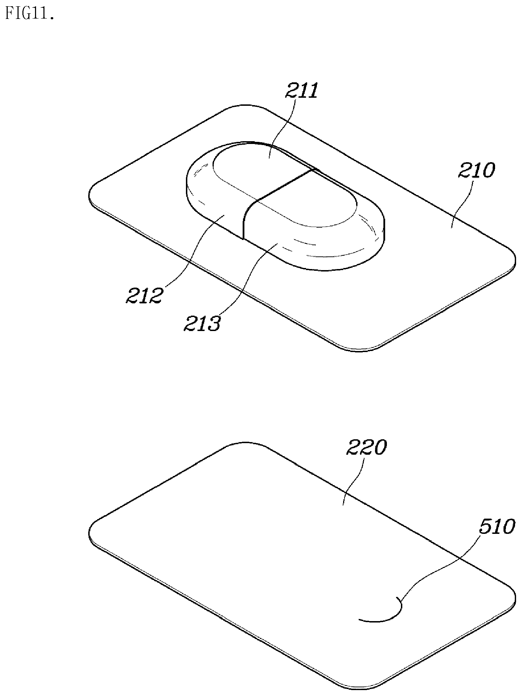

Hereinafter, a mask pack according to a third embodiment of the present invention will be described in detail with reference to FIGS. 11 to 15.

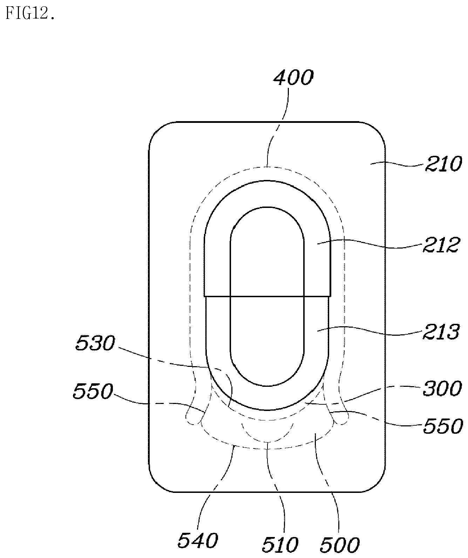

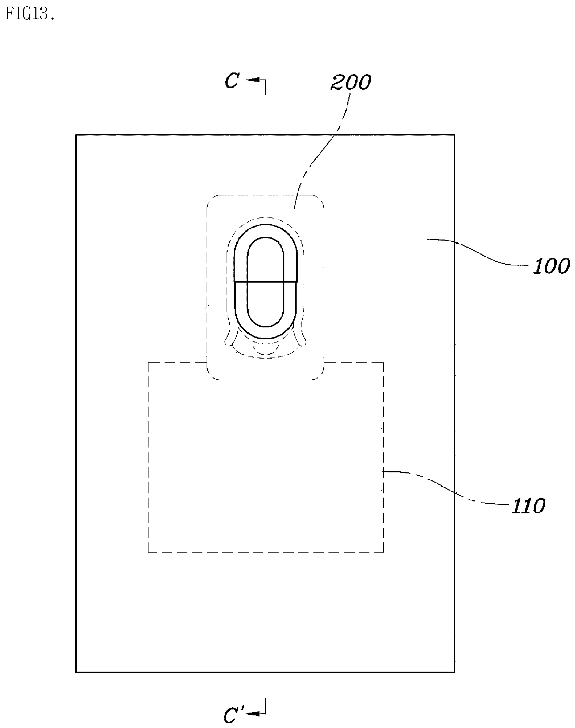

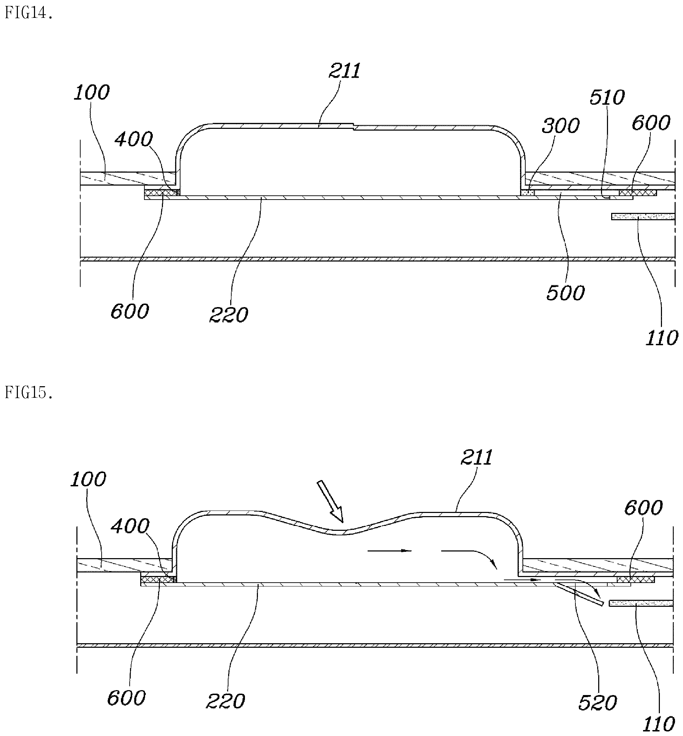

FIG. 11 is a perspective view of a mask pack according to the third embodiment of the present invention with a blister separated, FIG. 12 is a plan view showing the blister of the mask pack according to the third embodiment of the present invention, FIG. 13 is a view showing the blister inserted in a pouch of the mask pack according to the third embodiment of the present invention, FIG. 14 is a cross-sectional view taken along line C-C' of FIG. 13, and FIG. 15 is a view showing an example of actually using the mask pack according to the third embodiment of the present invention.

The mask pack according to the third embodiment of the present invention may include a pouch 100 that keeps a mask sheet 110 and a blister 200.

In detail, as shown in FIGS. 11 and 12, the blister 200 may have: a first adhesive section 300 bonded to a cover 220, as a section of the edge of a storing portion 211, to be detached when pressure over a predetermined magnitude is applied to the storing portion 211; a second adhesive section 400 bonded to the cover 220 more strongly than the first adhesive portion 300, as the other section except for the first adhesive section of the edge of the storing portion 211; a non-adhesive section 500 formed outside the first bonding section 300 and having a predetermined area where the body 210 and the cover 220 are not bonded to each other; a third adhesive section 600 bonding the body and the cover to each other outside the edge of the storing portion 211 and the non-adhesive section 500; and a crease 510 formed on the cover 220 at the non-adhesive section 500. The edges of a first end and a second end of the storing portion 211 may be formed in an arc shape.

In detail, the first adhesive section 300 may be bonded to the cover 220, as a section of the edge of the storing portion 211, and may have the same curvature as the edge of the second end of the storing portion 211. In detail, the first adhesive section 300 is formed between the body 210 and the cover 220, and in more detail, a section of the edge of the storing portion 211 of the body 210 may be physically attached to the cover 220 by an embossing structure. That is, at the first adhesive section 310, unlike the second adhesive section 400 to be described below, the body 210 and the cover 220 are not completely bonded to each other by an adhesive etc., but are physically coupled to each other by an embossing structure. As described above, since the body 210 and the cover 220 are physically coupled to each other at the first adhesive section 300, when pressure over a predetermined magnitude is applied to the storing portion 211, the body 210 and the cover 200 physically coupled to each other can be separated by the pressure.

The first adhesive section 300 prevents leakage of a cosmetic liquid out of the storing portion 211 and the adhesive strength at the first adhesive section 300 may depend on the area of the first adhesive section 300. For example, when the area of the first adhesive section 300 is too small, the physically coupled area by the embossing structure between the body 210 and the cover 220 is small, so the adhesive strength at the first adhesive section 300 may be small. Accordingly, even if small pressure is applied to the storing portion 211, the bonding at the first adhesive section 300 may be easily removed, so the cosmetic liquid in the storing portion 211 may be discharged. To prevent this problem, the first adhesive section 300 may be formed in an area having a predetermined size or more.

The second adhesive section 400 may be bonded to the cover 220 as the other section except for the first adhesive section 300 of the edge of the storing portion 211. The adhesive strength may be larger at the second adhesive section 400 than at the first adhesive section 300. In detail, at the second adhesive section 400, the body 210 and the cover 220 may be chemically attached to each other by an adhesive. That is, the body 210 and the cover 220 are chemically completely bonded to each other at the second adhesive section 400 by an adhesive to prevent the cosmetic liquid from being discharged out of the storing portion 211 though the second adhesive section 400 even if pressure over a predetermined magnitude is applied to the storing portion 211.

As described above, when pressure over a predetermined magnitude is applied to the storing portion 211, the cosmetic liquid is not discharged through the second adhesive section 400 and is discharged only through the first adhesive section 300 so that the cosmetic liquid is discharged only through a smaller area, so the cosmetic liquid can be discharge more quickly. The more quickly the cosmetic liquid is charged, the more uniformly the cosmetic liquid can be distributed to the bottom of the pouch of the mask pack. Accordingly, in the present invention, it is possible to enable the cosmetic liquid to uniformly permeate into the bottom of the mask sheet by maximally increasing the discharge speed of the cosmetic liquid.

The second adhesive section 400, as shown in FIG. 12, may extend to both sides of non-adhesive section 500 to be described below.

The non-adhesive section 500, as shown in FIG. 12, is formed outside the first adhesive section 300 and may have an inner end 530, an outer end 540, and both sides 550. The non-adhesive section 500 may be formed in a predetermined area, and the body 210 and the cover 220 are not bonded to each other at the non-adhesive section 500.

In detail, referring to FIG. 12, the inner end 530 may be spaced a predetermined distance from the edge of a second part of the storing portion 211, the outer end 540 may be spaced a predetermined distance from the inner end 530, and the both sides 550 may be formed inside the second adhesive section 400. That is, the non-adhesive section 500 is surrounded by the first adhesive section 300, the second adhesive section 400, and the third adhesive section 600 to be described below, and the body 210 and the cover 220 is not bonded to each other at the non-adhesive section 500. Accordingly, when pressure over a predetermined magnitude is applied to the storing portion 211, the first adhesive section 300 is detached, and the cosmetic liquid in the storing portion 211 is discharged, the cosmetic liquid collects in a predetermined area of the non-adhesive section 500 without being discharged through the second adhesive section 400 and the third adhesive section 600.

The crease 510 may be formed on the cover 220 at the non-adhesive section 500. In detail, the crease 410 may be formed in a curve shape convexly toward the outer end 540 of the non-adhesive section 500 at a predetermined distance from the first adhesive section 300.

When pressure over a predetermined magnitude is applied to the storing portion 211, the first adhesive section 300 is detached, and the cosmetic liquid in the storing portion 211 is discharged, the cover 220 is torn downward at the crease 510 by pressure of the cosmetic liquid. As the cover is torn downward at the crease 510, a discharge hole 520 is formed, as shown in FIG. 15.

As described above, since the crease 510 is formed in a curve shape convexly toward the outer end 540 in the present invention, the cover is more easily torn downward at the crease 510 when the cosmetic liquid in the storing portion 211 is discharged, whereby the discharge hole 520 is formed and the cosmetic liquid can be more smoothly discharged through a larger area, that is, the discharge hole 520. If the crease 510 is formed in straight light, the cover is not opened well at the crease 510, so the cosmetic liquid discharged out of the storing portion 211 may not smoothly flow into the pouch of the mask pack. In the present invention, this problem is solved by forming the crease 410 convexly toward the outer end 540.

The third adhesive section 600 is formed outside the edge of the storing portion 211 and the non-adhesive section 500, where the body 210 and the cover 220 are bonded to each other. That is, the third adhesive section 600 may be a section having a predetermined area outside the second adhesive section 400 and the outer end 540 of the non-adhesive section 500. Depending on embodiments, at the third adhesive section 600, the body 210 and the cover 220 may be physically coupled to each other by an embossing structure or may be chemically bonded to each other by an adhesive.

An example of actually using the mask pack according to the third embodiment of the present invention is described with reference to FIG. 15. When pressure over a predetermined magnitude is applied to the storing portion 211 exposed outside the pouch 100, the first adhesive section 300 is detached, and the cosmetic liquid in the storing portion 211 is discharged, the cover 220 is torn downward at the crease 510 and the discharge hole 520 is formed at the torn portion. Accordingly, the cosmetic liquid is discharged into the pouch 100 through the discharge hole 520, so the mask sheet can be soaked with the cosmetic liquid. According to the mask pack of the third embodiment of the present invention, as described above, a cosmetic liquid is separately kept in the separate blister, and when the storing portion exposed outside the pouch is pressed by force over a predetermined magnitude to use a facial mask, the body and the cover are detached at the first adhesive section and the cosmetic liquid in the storing portion is discharged. Further, the cosmetic liquid is discharged into the pouch through the discharge hole formed by the pressure of the discharged cosmetic liquid. Therefore, a user can simply mix and use the cosmetic liquid and the mask sheet and use a fresher and more sanitary facial mask, and accordingly, the skincare effect can be improved.

Meanwhile, in the mask packs according to embodiments of the present invention, the mask sheet 110 disposed in the pouch 100 may have been soaked with a basic cosmetic liquid. In this case, in order to use a facial mask, when the cosmetic liquid in the storing portion is discharged into the pouch in accordance with the simple methods described above, the cosmetic liquid in the storing portion permeates into the mask sheet soaked with a basic cosmetic liquid. Accordingly, it is possible to improve the skincare effect, using a chemical reaction of several different cosmetic liquids.

## Claims

1. A mask pack comprising:
a pouch keeping a mask sheet and having a hole at a side;
a blister including a body formed in a plate shape, having a storing portion protruding upward from the center portion to the outside through the hole of the pouch to keep a cosmetic liquid, and having the other portion fixed to an inner side of the pouch in the pouch, except for the storing portion, and a cover covering a bottom of the body to seal the storing portion and being torn or removed by external force so that the cosmetic liquid is discharged into the pouch and permeates into the mask sheet.

2. The mask pack of claim 1, wherein the cover is formed with a predetermined thickness and has a groove having a predetermined length in a width direction, wherein when external force over a predetermined magnitude is applied to both ends of the body such that the blister is bent, the groove on the cover is torn and the cosmetic liquid in the storing portion is discharged into the pouch, so the mask sheet is soaked with the cosmetic liquid.

3. The mask pack of claim 1, wherein the cover has: a sealing portion sealing the storing portion by covering the bottom of the body; and a pull extending from an end of the sealing portion.

4. The mask pack of claim 3, wherein the pull extends from an end of the sealing portion longer than a maximum length of the body.

5. The mask pack of claim 3, wherein a first end of the pull is bonded to the bottom of the body and a second end of the pull is folded to a second end of the sealing portion from the first end bonded to the bottom of the body.

6. The mask pack of claim 5, wherein a slit through which the second end of the pull is disposed is formed through a side of the pouch.

7. The mask pack of claim 6, wherein when the blister is fixed to the pouch, the second end of the pull is exposed to the outside through the slit of the pouch.

8. The mask pack of claim 5, wherein a slit through which the second end of the pull is disposed is formed through a side of the body.

9. The mask pack of claim 8, wherein when the hole of the pouch is larger than the storing portion of the blister and the blister is fixed to the pouch, the second end of the pull is exposed to the outside trough the slit of the body and the hole of the pouch.

10. The mask pack of claim 1, wherein the blister has:
a first adhesive section bonded to the cover, as a section of an edge of the storing portion, to be detached when pressure over a predetermined magnitude is applied to the storing portion;
a second adhesive section bonded to the cover more strongly than the first adhesive portion, as the other section except for the first adhesive section of the edge of the storing portion;
a non-adhesive section formed outside the first bonding section and having a predetermined area where the body and the cover are not bonded to each other;
a third adhesive section bonding the body and the cover to each other outside the edge of the storing portion and the non-adhesive section; and
a crease formed on the cover at the non-adhesive section.

11. The mask pack of claim 10, wherein the body and the cover are physically coupled to each other at the first adhesive section by an embossing structure.

12. The mask pack of claim 10, wherein the non-adhesive section has an inner end, an outer end, and a both sides.

13. The mask pack of claim 12, wherein the second adhesive section extends to the both sides of the non-adhesive section.

14. The mask pack of claim 12, wherein the inner end is spaced a predetermined distance from the edge of a second part of the storing portion, the outer end is spaced a predetermined distance from the inner end, and the both sides are formed inside the second adhesive section.

15. The mask pack of claim 10, wherein when pressure over a predetermined magnitude is applied to the storing portion, the first adhesive section is detached, and the cosmetic liquid in the storing portion is discharged, the cover is torn downward at the crease by pressure of the cosmetic liquid, a discharge hole is formed at the torn portion, and the cosmetic liquid is discharged into the pouch through the discharge hole and permeates into the mask sheet.
